# EUROPEAN PATENT APPLICATION

(11) **EP 4 617 359 A1**
(43) Date of publication of application: **17.09.2025**
(21) Application number: 23889122.0
(22) Date of filing: 07.11.2023
(51) Int. Cl.: C12N 1/16, A21D 8/04, C12R 1/865

(54) **SACCHAROMYCES CEREVISIAE FRUITFLY STRAIN CAPABLE OF MAINTAINING FERMENTATION POWER IN HIGH-SUGAR DOUGH**

(30) Priority: 07.11.2022 KR 20220147358
(71) Applicant: Le Pain Co., Ltd., Seoul 03021 (KR)
(72) Inventor: LIM, Tae Eon, Seoul 04711 (KR); LEE, Gye Joon, Pyeongtaek-si Gyeonggi-do 17813 (KR); BAEK, Jong Hyeon, Seoul 01683 (KR); LIM, Soo Jeong, Seoul (KR); KIM, Gye Ryeong, Seoul 01776 (KR)
(74) Representative: M. Zardi & Co S.A.
(86) International application number: PCT/KR2023/017799
(87) International publication number: WO 2024/101864

(57) **Abstract**

The present invention relates to a Saccharomyces cerevisiae FRUITFLY strain (Accession No. KCTC 15094BP) that maintains fermentative capacity in high sugar content dough. The strain maintains fermentative capacity in dough with high sugar content of up to 54 Brix and the fermentative capacity of the strain is enhanced in high sugar content dough when sodium L-ascorbate is added to the dough.

## Description

### [TECHNICAL FIELD]

The present invention relates to a Saccharomyces cerevisiae FRUITFLY strain (Accession No. KCTC 15094BP) that maintains fermentative capacity in high sugar content dough.

### [BACKGROUND OF THE INVENTION]

Various compositions of wheat dough are used in bread, ranging from breads with low quantity of sucrose or no sucrose, such as French bread and white bread, to breads with high quantity of sucrose. Bread yeast strains used for bread-making also vary in their fermentative capacity depending on the sugar content of the dough. For doughs with high sucrose content, yeast strains with high sugar tolerance are typically selected.

Sugar tolerance in yeast discussed traditionally means resistance to sucrose i.e. sucrose tolerance. Studies on the sucrose tolerance of baker's yeast have been conducted for a long time with reports indicating a correlation with the activity of invertase. Invertase is an extracellular enzyme that hydrolyzes the disaccharide sucrose into its constituent monosaccharides, glucose and fructose.

Sucrose is broken down into monosaccharides by invertase outside the yeast cell and then the resulting sugars are taken up into the cell and utilized as a nutrient source. In yeast strains with high invertase activity, the rapid hydrolysis of sucrose leads to an increase in osmotic pressure around the yeast cells in the dough, which in turn suppresses yeast fermentation. As a result, there is a negative correlation between invertase activity and sucrose tolerance, and currently, yeast strains used in snack have typically low invertase activity.

As such, previous reports on sucrose tolerance have either suggested that invertase activity plays a role in sucrose tolerance, or that the contribution of invertase is limited. In other words, sucrose tolerance can be considered as a composite trait involving both invertase activity and osmotic pressure resistance. Most conventional breeding approaches to enhance sucrose tolerance have focused on reducing invertase activity. Furthermore, the level of sucrose tolerance achieved under practical fermentative capacity has typically been limited to up to 30% sucrose concentration.

### [DETAILED DESCRIPTION OF THE INVENTION]

### [PROBLEMS TO BE SOLVED]

The present invention provides a Saccharomyces cerevisiae FRUITFLY strain (Accession No. KCTC 15094BP) that maintains fermentative capacity in high sugar content dough.

### [SOLUTION]

According to one embodiment of the present invention, the present invention provides a *Saccharomyces cerevisiae* FRUITFLY strain (Accession No. KCTC 15094BP) that maintains fermentation power in high sugar content dough.

The strain maintains fermentative capacity in dough with high sugar content of up to 54 Brix.

The fermentative capacity of the strain is enhanced in high sugar content dough when sodium L-ascorbate is added to the dough.

The dough includes the strain, wheat flour, sugar, salt and water.

The dough comprises, per 100 parts by weight of wheat flour, 10 to 50 parts by weight of sugar, 1 to 5 parts by weight of salt, 1 to 5 parts by weight of the strain and 30 to 60 parts by weight of water.

The sodium L-ascorbate is included in an amount of 0.01 to 0.2 parts by weight per 100 parts by weight of wheat flour.

According to another embodiment of the present invention, the present invention provides a bread produced using the strain.

### [BENEFITS OF THE INVENTION]

The *Saccharomyces cerevisiae* FRUITFLY strain (Accession No. KCTC 15094BP) according to the present invention maintains fermentative capacity in doughs with high sugar content of up to 54 Brix, and the fermentative capacity of the strain is enhanced in high sugar content dough when sodium L-ascorbate is added to the dough.

### [BREIF DESCRIPTION OF DRAWINGS]

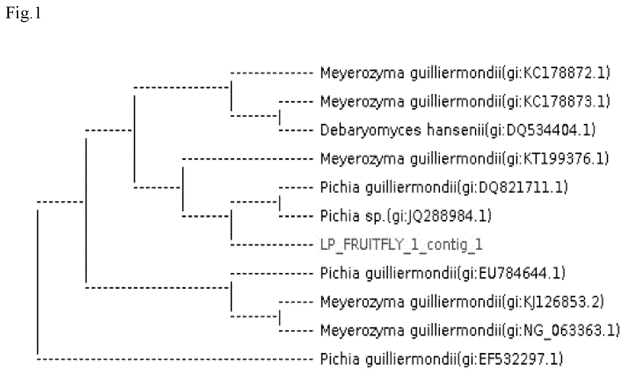

FIG. 1 a diagram showing a phylogenetic tree of the *Saccharomyces cerevisiae* FRUITFLY strain (Accession No. KCTC 15094BP) of the present invention.
FIG. 2 depicts the measurement result of sugar tolerance of the *Saccharomyces cerevisiae* FRUITFLY strain (Accession No. KCTC 15094BP) in culture medium with high sugar content.

### [EMBODIMENTS OF THE INVENTION]

In the present specification, when a component is described as being "on" another component, it is to be understood that this includes both cases where the components are in direct contact and cases where one or more additional components are interposed between them.

In the present specification, when a part is described as "comprising" a certain component, unless otherwise specified, this does not exclude the presence of other components, and the part may further comprise additional components.

According to one embodiment of the present invention, the present invention provides a *Saccharomyces cerevisiae* FRUITFLY strain (Accession No. KCTC 15094BP).

The *Saccharomyces cerevisiae* FRUITFLY strain (Accession No. KCTC 15094BP) of the present invention maintained fermentative capacity in high sugar content dough up to 54° Brix. Furthermore, the strain exhibited enhanced fermentative capacity in high sugar content dough when sodium L-ascorbate is added to the dough.

According to the inventor's experiments, while the fermentative capacity of the strain was reduced in dough with a sugar content exceeding 54 Brix, the addition of sodium L-ascorbate resulted in improved fermentative capacity.

The dough comprised, per 100 parts by weight of wheat flour, 10 to 50 parts by weight of sugar, 1 to 5 parts by weight of salt, 1 to 5 parts by weight of the strain and 30 to 60 parts by weight of water.

The sodium L-ascorbate was included in an amount of 0.01 to 0.2 parts by weight per 100 parts by weight of wheat flour.

The strain maintained fermentative capacity in high sugar content dough. Then the fermentative capacity of the dough can be improved and thereby result in softer bread when making bread using a dough with high sugar content.

Based on identification results, the strain of the present invention belongs to the species *Saccharomyces cerevisiae.* In the present invention, the strain has been designated as *Saccharomyces cerevisiae* FRUITFLY, and was deposited with Korea Research Institute of Bioscience and Biotechnology (KRIBB) on September 21, 2022, under the accession number KCTC 15094BP.

### [EXAMPLES]

### 1. Isolation of the strain

To prepare a liquid medium, 0.5% yeast extract, 0.5% malt extract, 0.3% peptone, and 0.5% dextrose were added to 500 mL of sterilized water. The medium was sterilized using an autoclave at 121°C, 1 atm (15 psi) for 15 minutes. Freshly collected wildflowers were added to the liquid medium in an amount equivalent to 5% of the medium volume, mixed thoroughly, and statically incubated at 30°C for 48 hours.

To prepare a solid medium, 3 g yeast extract, 3 g malt extract, 3 g peptone, 300 g sucrose and 15 g agar were added to 1 L of sterilized water. The solid medium was sterilized using an autoclave at 121°C, 1 atm (15 psi) for 15 minutes. Microorganisms cultured in the liquid medium were transferred to the solid medium using a inoculation wand, and streaked on the surface of the plate, and colonies were isolated.

A single colony was then cultured into 500 mL of sterilized YM liquid medium, strains exhibiting excellent fermentative capacity in high sugar content dough of 40 Brix were selected. The inventor performed 18S rRNA identification on the isolated strain, which was identified as *Saccharomyces cerevisiae.*

The inventor designated the strain as *Saccharomyces cerevisiae* FRUITFLY and deposited it with the Korea Research Institute of Bioscience and Biotechnology (KRIBB) on September 21, 2022, under the accession number KCTC 15094BP.

The 18S rRNA sequence of *Saccharomyces cerevisiae* FRUITFLY is as follows:

[SEQ ID NO.1]
GTCCTGCATGTCTAGTATAAGCATTTATACAGTGAAACTGCGAATGGCTCAT
TAAATCAGTTATCGTTTATTTGATAGTACCTTACTACTTGGATAACCGTGGT
AATTCTAGAGCTAATACATGCTTAAAATCCCGACTGTTTGGAAGGGATGTAT
TTATTAGATAAAAAATCAATGCCTTCGGGCTCTTTGATGATTCATAATAACT
TTTCGAATCGCATGGCCTTGTGCTGGCGATGGTTCATTCAAATTTCTGCCCT
ATCAACTTTCGATGGTAGGATAGTGGCCTACCATGGTTTCAACGGGTAACG
GGGAATAAGGGTTCGATTCCGGAGAGGGAGCCTGAGAAACGGCTACCACAT
CCAAGGAAGGCAGCAGGCGCGCAAATTACCCAATCCCGACACGGGGAGGT
AGTGACAATAAATAACGATACAGGGCCCTTTTGGGTCTTGTAATTGGAATG
AGTACAATGTAAATACCTTAACGAGGAACAATTGGAGGGCAAGTCTGGTGC
CAGCAGCCGCGGTAATTCCAGCTCCAAAAGCGTATATTAAAGTTGTTGCAG
TTAAAAAGCTCGTAGTTGAACCTTGGGCTTGGCTGGCCGGTCCATCTTTTTG
ATGCGTACTGGACCCAGCCGAGCCTTTCCTTCTGGGTAGCCATTTATGGCGA
ACCAGGACTTTTACTTTGAAAAAATTAGAGTGTTCAAAGCAGGCCTTTGCTC
GAATATATTAGCATGGAATAATAGAATAGGACGTTATGGTTCTATTTTGTTG
GTTTCTAGGACCATCGTAATGATTAATAGGGACGGTCGGGGGTATCAGTATT

CAGTTGTCAGAGGTGAAATTCTTGGATTTACTGAAGACTAACTACTGCGAA
AGCATTAACCAAGGACGTTTTCATTAATCAAGAACGAAAGTTAGGGGATCG
AAGATGATCAGATACCCGTCGTAGTCTTAACCATAACTATGCGACAAGGGA
ACCGGTTGTTGTTCTTTTATTGACGCAATCGGCACTTACAGAATCAAATCTT
GGTCTGGGGGGGGGATAATGTTCGCCAGGGTGAAACTTAAGGAATTGTCGG
AGGGCCCCCCCGGAGGGGAGCGGCGGGTTATTTGTTCCCCCGGGGAAACCC
CCCGGCCACCCACAAGGATGTGAGAATGAAGCTTTTGTTTTTTGTGGGGGG
GGGGGGGGGGGGCGCCCCCCCGGGGGGGGGGGGTGGGTTTGTGTGTGTAA
GAGAGAAAAACAAAAACCAAAAAAGAGGGGGGGTGGGGGGGTGTTTTTTT
TAAAAAAAAAATATTTTCTAAATAAAAAAAAAAAAAAAAACCCCTCTTTTT
CCCCCCCCCCCCCCCCCCCCCCATCCCTTGGTGTTTATATTTTTTCTGGTGGG
GGTTTTTTTTATTATCAAAAAAAAAAAAAATTTCTTTTCCCCCCCA

### [EXPERIMENTS]

### 1. Measurement of sugar tolerance of the strain

3 g of yeast extract, 3 g of malt extract, 3 g of peptone, sucrose (300 g, 400 g, 500 g, or 600 g), and 15 g of agar were added to 1,000 mL of sterilized water to prepare medium. The medium was boiled at over 100°C for 1 minute, and mixed thoroughly, and then sterilized using an autoclave at 121°C and 1 atm (15 psi) for 15 minutes. After sterilization, 25 g of the medium was poured into each Petri dish and solidified.

Commercial baker's yeast (Jenico baker's yeast), high-sugar tolerant yeast (Lesaffre), and the *Saccharomyces cerevisiae* FRUITFLY strain of the present invention were each cultured in YM liquid medium for 12 hours, and were then streaked onto the high-sugar solid medium, and cultured at 30°C for 24 and 48 hours.

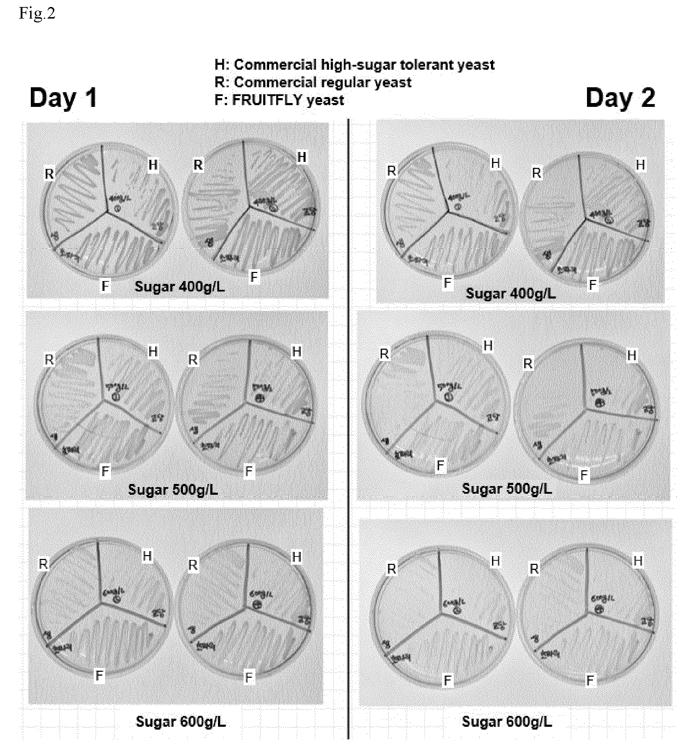

As shown in FIG. 2, the *Saccharomyces cerevisiae* FRUITFLY strain of the present invention was able to grow normally even on high-sugar medium containing 60% (600 g/L) sucrose.

In contrast, both the commercial baker's yeast and the high-sugar tolerant yeast did not exhibit normal growth under the same conditions. The results indicate that the *Saccharomyces cerevisiae* FRUITFLY strain possesses superior sugar tolerance compared to conventional high-sugar tolerant yeast.

### 2. Measurement of fermentative capacity of the strain in high sugar content dough

The isolated *Saccharomyces cerevisiae* FRUITFLY strain was inoculated into sterilized YM liquid medium and cultured for 16 hours. After culture, the cells were harvested by centrifugation, followed by dehydration to obtain cell masses with a moisture content of 65-70%.

Subsequently, dough was prepared according to the composition shown in [Table 1], divided into 30 g portions, and the total gas production during fermentation at 30°C for 120 minutes was measured using a Fermograph.

A dough was prepared by mixing 100 g of flour, 2 g of salt, 2 g of yeast, and 50 g of water, with adding varying amounts of sugar. Consequently, the sugar concentration was between 10 and 60 Brix.

**[Table 1]**

| | 10 Brix | 20 Brix | 30 Brix | 40 Brix | 50 Brix | 60 Brix |
|---|---|---|---|---|---|---|
| Commercial yeast | 85 | 63 | 30 | 13 | 11 | 5 |
| Fruit fly | 102 | 100 | 95 | 92 | 90 | 51 |

The *Saccharomyces cerevisiae* FRUITFLY strain of the present invention exhibited superior fermentative capacity in all doughs compared to commercial yeast. As shown in [Table 1], it was observed that the fermentation power of commercial yeast decreased as the sucrose concentration increased from 10 Brix to 60 Brix.

In contrast, the *Saccharomyces cerevisiae* FRUITFLY strain demonstrated superior fermentative capacity over the commercial yeast at all concentrations, including at a sucrose concentration of 50 Brix. However, at 60 Brix, the fermentative capacity significantly decreased.

### 3. Measurement of maximum concentration

The inventor aimed to determine the maximum sugar concentration at which the *Saccharomyces cerevisiae* FRUITFLY strain can maintain fermentative capacity. Doughs with sucrose concentrations of 50, 52, 54, 56, 58, and 60 Brix were prepared, divided into 30 g portions, and total gas production during fermentation at 30°C for 120 minutes was measured using a Fermograph.

**[Table 2]**

| | 50 Brix | 52 Brix | 54 Brix | 56 Brix | 58 Brix | 60 Brix |
|---|---|---|---|---|---|---|
| *S. cerevisiae* FRUITFLY | 92 | 90 | 85 | 64 | 58 | 52 |

The fermentative capacity of the *Saccharomyces cerevisiae* FRUITFLY strain was measured in more detailed increments, and it was confirmed that the strain maintained sufficient fermentative capacity for producing bread with up to 54 Brix. However, after the point, the fermentative capacity rapidly decreased.

### 4. Measurement of fermentative capacity of the strain in dough with added sodium L-ascorbate

The inventor screened substances that could enhance the sugar resistance of the isolated *Saccharomyces cerevisiae* FRUITFLY strain.

Various food additives were mixed into a dough with 60 Brix, and dough was prepared. The dough was divided into 30 g portions, and total gas production during fermentation at 30°C for 120 minutes was measured using a Fermograph. Each substance was added at a concentration of 0.1 g.

**[Table 3]**

| | sodium L-ascorbate | Sodium gluconate | Monosodium L-glutamate |
|---|---|---|---|
| total gas production | 95 | 45 | 47 |

According to the total gas production shown in Table 3, it was confirmed that the fermentative capacity of the isolated *Saccharomyces cerevisiae* FRUITFLY strain was improved when sodium L-ascorbate was added to the dough with a sugar concentration of 60° Brix. However, when sodium gluconate or monosodium L-glutamate was added, the total gas production did not increase, indicating that the additives did not enhance the fermentative capacity of the *Saccharomyces cerevisiae* FRUITFLY strain in high sugar content dough.

### [Depositary Information]

Depositary Institution: Korean Collection for Type Cultures (KCTC), Korea Research Institute of Bioscience and Biotechnology
Accession Number: KCTC 15094BP
Date of Deposit: Sep. 21, 2022

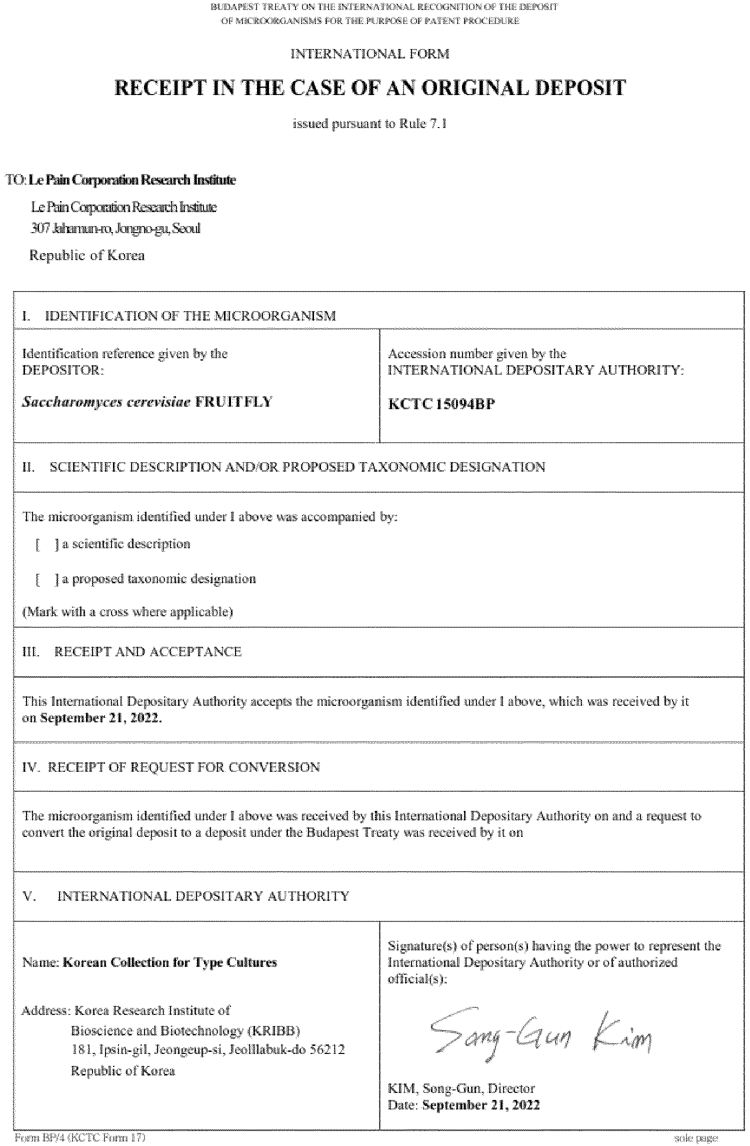
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Le Pain Corporation Research Institute
Le Pain Corporation Research Institute
307 Jahamun-ro, Jongno-gu, Seoul
Republic of Korea

I. IDENTIFICATION OF THE MICROORGANISM
Identification reference given by the
DEPOSITOR:

Saccharomyces cerevisiae FRUITFLY | Accession number given by the
INTERNATIONAL DEPOSITARY AUTHORITY:

KCTC 15094BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on September 21, 2022.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: September 21, 2022
Form BP/4 (KCTC Form 17) sole page

## Claims

1. *Saccharomyces cerevisiae* FRUITFLY strain (Accession No. KCTC 15094BP), wherein the strain maintains fermentative capacity in high sugar content dough, and wherein fermentative capacity of the strain is enhanced in high sugar content dough when sodium L-ascorbate is added to the dough.

2. The strain of Claim 1, wherein the strain maintains fermentative capacity in dough with high sugar content of up to 54 Brix.

3. The strain of Claim 1, wherein the dough includes the strain, wheat flour, sugar, salt and water.

4. The strain of Claim 3, wherein the dough comprises, per 100 parts by weight of wheat flour, 10 to 50 parts by weight of sugar, 1 to 5 parts by weight of salt, 1 to 5 parts by weight of the strain and 30 to 60 parts by weight of water.

5. A bread product prepared using the strain according to any one of claims 1 to 4.
